(19) Europäisches Patentamt
European Patent Office
Office européen des brevets

(11) Veröffentlichungsnummer: **0 333 004**
**A2**

(12) **EUROPÄISCHE PATENTANMELDUNG**

(21) Anmeldenummer: 89104057.8

(51) Int. Cl.4: C07C 113/04 , G03C 1/54

(22) Anmeldetag: 08.03.89

(30) Priorität: 15.03.88 DE 3808590

(43) Veröffentlichungstag der Anmeldung: 20.09.89 Patentblatt 89/38

(84) Benannte Vertragsstaaten: AT BE CH DE ES FR GB IT LI NL SE

(71) Anmelder: HOECHST AKTIENGESELLSCHAFT
Postfach 80 03 20
D-6230 Frankfurt am Main 80(DE)

(72) Erfinder: Siegel, Herbert, Dr. Dipl.-Chem.
Am alten Birnbaum 10 a
D-6238 Hofheim(DE)
Erfinder: Erdmann, Fritz, Dr. Dipl.-Chem.
Am Steinberg 3
D-6228 Eltville 4(DE)
Erfinder: Lutz, Walter, Dr. Dipl.-Chem.
Herderstrasse 21
D-6203 Hochheim(DE)

(54) **Lichtempfindliche Diazoniumverbindung, Verfahren zu ihrer Herstellung und ihre Verwendung.**

(57) Die Erfindung betrifft eine neue lichtempfindliche Diazoniumverbindung der allgemeinen Formel I

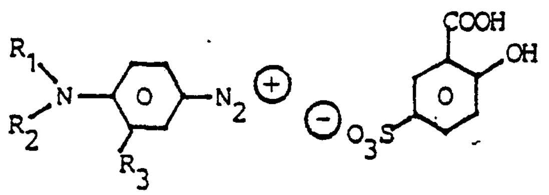

I

in der $R_1$ und $R_2$ gleich oder verschieden sind und
$R_1$ Methyl, Ethyl oder 2-Hydroxyethyl und
$R_2$ Methyl oder Ethyl oder
$R_1$ und $R_2$ gemeinsam eine $(CH_2)_n$ Gruppierung über dem Stickstoffatom, an das sie gebunden sind, mit n gleich 4 oder 5 und
$R_3$ Wasserstoff oder Methyl bedeuten. Die Erfindung betrifft ferner ein Verfahren zur Herstellung der lichtempfindlichen Diazoniumverbindung. Die Verbindungen finden insbesondere in lichtempfindlichen Diazotypiematerialien Verwendung.

EP 0 333 004 A2

Xerox Copy Centre

## Lichtempfindliche Diazoniumverbindung, Verfahren zu ihrer Herstellung und ihre Verwendung

Die Erfindung betrifft neue lichtempfindliche Diazoniumverbindungen, ein Verfahren zu ihrer Herstellung und die Verwendung in lichtempfindlichen Diazotypiematerialien.

Aus der US-Patentschrift Nr. 3,522,048 sind p-Chlorbenzolsulfonate von Diazoniumsalzen bekannt. Sie sind in 4-Stellung zur Diazoniumgruppe durch einen heterocyclischen basischen Rest substituiert und zur Herstellung wärmeentwickelbarer Diazotypiematerialien mit großer Verarbeitungsgeschwindigkeit geeignet. Lichtempfindliche Gemische, die diese Diazoniumsalze enthalten sind zwar gut thermisch stabil, die Diazoniumsalze selbst lösen sich jedoch nicht ausreichend in wäßrigen Sensibilisierungszubereitungen.

Aus DE-OS 33 08 395, entsprechend US-Patentschrift Nr. 4,590,143, sind Diazoniumverbindungen bekannt, die als Benzol-bzw. Toluolsulfonat vorliegen. Die Diazoniumverbindungen leiten sich von p-Phenylendiamin ab, tragen einen heterocyclischen Rest in 4-Stellung und sind in 2- und 5-Stellung durch Ethergruppen substituiert.

Aus DE-PS 32 02 208, entsprechend US-Patentschrift Nr. 4,403,028, ist bekannt, Diazoniumsalze in der Form von Sulfoisophthalaten abzuscheiden.

Bekannt ist auch die Verwendung von Benzoldiazoniumsalzen als lichtempfindliche Substanzen in Diazotypiematerialien. Zur Stabilisierung der Salze werden in der Regel Metallsalze, wie Zink-, Cadmium- oder Quecksilberchlorid und andere Schwermetallverbindungen, sowie Fluoride, wie $BF_4^-$, $PF_6^-$, $SbF_6^-$, oder $AsF_6^-$ eingesetzt.

Diese Abscheidungsformen sind zwar gut wasserlöslich, sie haben jedoch Nachteile bei ihrer Herstellung, ihrer Lagerung oder bei ihrer Handhabung.

Sie besitzen meist eine weniger gute thermische Beständigkeit, woraus hohe Sicherheitsvorkehrungen für Lagerung und Transport resultieren. Weiterhin sind sie teilweise wassergefährdend wegen des Gehalts an Schwermetall oder an Fluorid. Außerdem können sie oft nur in mäßigen Ausbeuten hergestellt werden, weil die Salze stark wasserlöslich sind. Hieraus ergeben sich unverhältnismäßig hohe Kosten für die erforderlichen Maßnahmen zur Entfernung der anfallenden Schwermetalle und Fluoride im Abwasser. Zusätzlich fallen im Herstellungsprozeß der bekannten Diazoniumsalze große Mengen an konzentrierter salzhaltiger z.B. Natriumchlorid-haltiger, Mutterlauge an, wodurch die Salzfracht der Abwässer in unerwünschter Weise erhöht wird.

Die Abscheidbarkeit und Brauchbarkeit von Benzoldiazoniumsalzen ist sehr stark abhängig von der Struktur des Benzoldiazoniumkations und des zur Abscheidung verwendeten Anions. Anionen, wie Chlorionen, führen zum Beispiel in der Regel zu einer Explosionsgefährlichkeit. Anionen, wie die der oben genannten Sulfonsäuren, vermindern stark die Wasserlöslichkeit oder führen nicht zu reinen Abscheidungsformen.

Es war Aufgabe der vorliegenden Erfindung eine Abscheidungsform für lichtempfindliche Diazoniumsalze anzugeben, bei der diese Nachteile bekannter Abscheidungsformen vermieden und gleichzeitig vergleichbare oder bessere anwendungstechnische Eigenschaften erzielt werden. Aufgabe der Erfindung war es auch die Diazoniumsalze in einfacher Weise und in großer Ausbeute herzustellen.

Die Aufgabe wird gelöst durch eine lichtempfindliche Diazoniumverbindung der allgemeinen Formel I

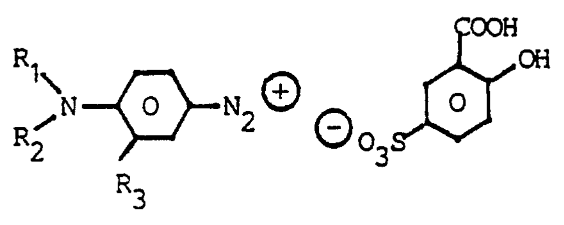

I

in der $R_1$ und $R_2$ gleich oder verschieden sind und

$R_1$ Methyl, Ethyl oder 2-Hydroxyethyl und

$R_2$ Methyl oder Ethyl oder

$R_1$ und $R_2$ gemeinsam eine $(CH_2)_n$ Gruppierung über dem Stickstoffatom, an das sie gebunden sind, mit n gleich 4 oder 5 und

$R_3$ Wasserstoff oder Methyl bedeuten. In bevorzugten Ausführungsformen bedeuten $R_1$, $R_2$ Methyl und $R_3$ Wasserstoff oder $R_1$ und $R_2$ zusammen $-(CH_2)_4-$ und $R_3$ Methyl.

Die erfindungsgemäßen Diazoniumverbindungen besitzen eine sehr gute Wärmebeständigkeit. Ihre

besonderen Vorteile gehen aus einem Vergleich mit üblichen Zinkchlorid-stabilisierten Diazoniumsalzen vergleichbarer Substituenten hervor.

Tabelle

Sicherheitskenndaten für stabilisierte Benzoldiazonium-Abscheidungsformen der allgemeinen Formel I

| Kriterium | $ZnCl_2$ stabilisiert | | Sulfosalicylat | |
|---|---|---|---|---|
| | $R_1=R_2=CH_3$ $R_3$ = H | $R_1, R_2=(CH_2)_4$ $R_3$ = $CH_3$ | $R_1=R_2=CH_3$ $R_3$ = H | $R_1, R_2=(CH_2)_4$ $R_3$ = $CH_3$ |
| Brennziffer (BZ) | 3 | 5 | 3 | 3 |
| Staubexplosionsklasse (ST) | 0 | 1 | 1 | 1 |
| Selbstentzündung (SE) °C | 120 | 120 | 150 | 120 |
| Exotherme Zersetzung (EZ) °C | 100 | 110 | 140 | 120 |
| Differentialthermoanalyse (DTA) kJ/kg | -720 | -450 | -490 | -400 |
| schlagempfindlich | nein | ja | nein | nein |
| explosionsgefährlich | nein | nein | nein | nein |

Im Vergleich zu dem Zinkchlorid-Doppelsalz ist die erfindungsgemäße Abscheidungsform mit $R_1$ und $R_2$ als Methyl und $R_3$ als Wasserstoff bei gleicher Brennbarkeit wesentlich stabiler. Die exotherme Zersetzung beginnt bei 140 °C und liegt damit um 40 °C höher als bei dem entsprechenden Zinkchlorid-Doppelsalz. Die Selbstentzündung beginnt bei 150 °C und damit erst bei wesentlich höherer Temperatur.

Die die Heftigkeit der Zersetzung charakterisierende Differential-Thermoanalyse weist mit -490 kJ/kg ebenfalls einen günstigeren Wert auf.

Ähnliche Befunde ergeben sich für die erfindungsgemäßen Abscheidungsformen mit $R_1$ und $R_2$ als $-(CH_2)_4-$ und $R_3$ als Methyl, wie dies der Tabelle zu entnehmen ist.

Die erfindungsgemäße Abscheidungsform ist weiterhin auch ökologisch vorteilhafter gegenüber den bekannten Schwermetall-oder Fluorid-stabilisierten Abscheidungsformen, da die Sulfosalicylsäure mit einem $LD_{50}$-Wert (LD - Letale Dosis) von 2450 mg/kg als unbedenklich eingestuft werden kann.

Ein weiterer großer Vorteil der erfindungsgemäßen Abscheidungsform betrifft das zugehörige Herstellungsverfahren. Die vorliegende Erfindung ist deshalb auch auf ein Verfahren zur Herstellung der Diazoniumverbindung der allgemeinen Formel I gerichtet, das dadurch gekennzeichnet ist, daß man eine Verbindung der allgemeinen Formel II

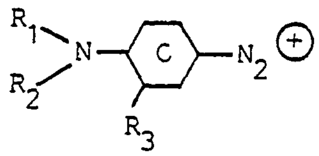

$X^{\ominus}$ II

worin $R_1$, $R_2$ und $R_3$ die angegebenen Bedeutungen haben und X das Anion einer starken Säure ist, mit einer Verbindung der allgemeinen Formel III

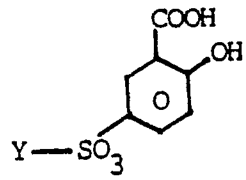

III

wobei Y ausgewählt ist aus der Gruppe Wasserstoff und Salz bildendes Metallion, in an sich bekannter Weise umsetzt und isoliert.

Hierdurch erhält man das erfindungsgemäße Diazoniumsulfo salicylat in wesentlich größerer Ausbeute und bei der Produktion fallen wesentlich geringere Abfallmengen an als in den bekannten vergleichbaren Fällen. Als unerwünschten Abfall erhält man bei der Produktion der dem Stand der Technik entsprechenden Schwermetall-stabilisierten Diazoniumsalze Mutterlaugen, die hohe Konzentrationen an Schwermetallen aufweisen. So sind z.B. Zink-haltige Abwässer bakterientoxisch und das enthaltene Zink muß daher in einem aufwendigen Reinigungsverfahren z.B. als Zinkhydroxid ausgefällt und abfiltriert werden. Wegen des amphoteren Charakters von Zinkhydroxid und der oftmals schlecht filtrierbaren Fällung stellt das Erreichen von für biologische Abwasserreinigungsanlagen tolerierbaren Restzinkgehalten im Abwasser ein erhebliches Problem dar. Der anfallende Zinkhydroxidschlamm muß anschließend einer Sondermülldeponie zugeführt werden. Dies stellt einen zusätzlichen Aufwand des herkömmlichen Herstellungsverfahrens dar. Hinzu kommt weiterhin, daß man, um gute Ausbeuten an Zink-stabilisiertem Diazoniumsalz zu erhalten, mit großen Mengen Salz, z.B. Kochsalz, aussalzen muß. Dies führt zu einer erheblichen Salzfracht und damit zu einer weiteren unerwünschten Belastung des Abwassers.

Ein besonderer Vorteil des erfindungsgemäßen Verfahrens ist außerdem darin zu sehen, daß man die Verbindungen der allgemeinen Formel I in sehr guten Ausbeuten aus wäßriger Lösung erhält. Ein Arbeiten mit organischen Lösungsmitteln, wie es gelegentlich zur Isolierung von Diazoniumsalzen in Substanz zur Erzielung guter Ausbeuten erforderlich ist, ist weder während der Diazotierung noch zur Ausfällung der Verbindungen der Formel I notwendig. Da die Produkte wasserfeucht anfallen, sind weder bei der Isolierung noch bei der Trocknung besondere Sicherheitsmaßnahmen zur Vermeidung zündfähiger Lösungsmittel/Luftgemische erforderlich.

Die bekannten Abscheidungsformen als Salze organischer Sulfonsäuren, wie Sulfoisophthalate, stellen zwar bezüglich Sicherheit und Umweltverträglichkeit gegenüber den beschriebenen Abscheidungsformen einen gewissen Fortschritt dar, ihre Löslichkeit in den bei der Herstellung von Diazotypiematerialien gebräuchlichen Lösungsmitteln ist jedoch gering, so daß ihre Verarbeitung hierdurch erschwert wird.

So beträgt die Löslichkeit in der Form des 4-N,N-Dimethylaminobenzoldiazonium-sulfoisophthalats in Wasser bei 20 °C 3,8 g/l, wogegen die Verbindung der Formel I mit $R_1 = R_2 = CH_3$ und $R_3$ Wasserstoff sich zu 10,4 g/l bei 20 °C löst. Bei den bekannten Sulfoisophtalaten müssen daher zur besseren Lösung Löslichkeit verleihende Verbindungen zugesetzt werden.

Die erfindungsgemäßen Verbindungen der Formel I sind aber nicht nur in Wasser, sondern auch in den für ihre Verarbeitung wichtigen organischen Lösungsmitteln, bzw. Lösungsmittelgemischen ausreichend löslich. Die Verbindungen der Formel I haben auch bei der Verwendung organischer Lösungsmittel den Vorteil, daß auf lösevermittelnde Zusätze verzichtet werden kann.

Das erfindungsgemäße Diazoniumsalz wird hergestellt, indem man zunächst eine Lösung des p-Aminobenzoldiazoniumions der allgemeinen Formel II herstellt und man das Diazoniumion anschließend mit Sulfosalicylsäure oder ihrem Ammonium-, Alkali- oder Erdalkali-Salz ausfällt. Als Lösungsmittel für diese Umsetzung dient vorzugsweise Wasser. Das Arbeiten mit organischen Lösungsmitteln oder mit Zusätzen organischer Lösungsmittel ist in der Regel nicht notwendig.

Die Lösung des p-Aminobenzoldiazoniumions der allgemeinen Formel II erhält man in an sich bekannter Weise durch Diazotierung des entsprechenden p-Aminoanilins in starker Mineralsäure, wie

Salzsäure oder Schwefelsäure, bei 0 - 40 °C, oder man setzt aus einem Schwermetall-stabilisierten Komplex durch Entfernen des Metalls das Diazoniumchlorid frei.

Die Ausfällung der beanspruchten Abscheidungsform geschieht wie folgt: Zur Lösung des p-Aminobenzoldiazoniumchlorids der allgemeinen Formel II wird unter Rühren Sulfosalicylsäure oder ihr Salz in fester Form oder als Lösung in einem geeigneten Lösungsmittel, vorzugsweise Wasser zugesetzt. Das molare Mengenverhältnis von Diazoniumsalz zu Sulfosalicylsäure liegt bei etwa 1: (1 bis 2), vorzugsweise bei 1: (1,05 bis 1,1). Meist reicht ein kleiner Überschuß an Fällmittel. Dieser hängt von der Konzentration der Diazoniumsalzlösung ab. Arbeitet man z.B. in 0,5 molarer Lösung, so genügt ein Überschuß von 5-10 %, in verdünnten Lösungen sind bis zu 100 % erforderlich. Die Temperatur während der Ausfällung beträgt vorzugsweise 20-30 °C, der pH-Wert kann zwischen 0 und 7 liegen. Anschließend kühlt man zur Vervollständigung der Fällung auf 0-5 °C ab und isoliert das Salz durch Filtrieren oder Zentrifugieren.

Die Ausfällung erfolgt im allgemeinen bereits während der Zugabe von Sulfosalicylsäure, so daß eine Nachrührzeit während des Abkühlens von weniger als zwei Stunden genügt.

Besonders bevorzugt wird ein Verfahren zur Herstellung der erfindungsgemäßen Verbindung, bei dem eine 0,5 molare Lösung des Benzoldiazoniumchlorids durch Diazotierung des entsprechenden p-Aminoanilins in wässriger Salzsäure bei 20-40 °C hergestellt wird. Zu dieser Lösung wird anschließend bei 20-25 °C feste Sulfosalicylsäure zugegeben, wobei die Zugabe in einem Zeitraum von wenigen Minuten erfolgen kann. Das erfindungsgemäße p-Aminobenzoldiazoniumsulfosalicylat fällt bereits während der Zugabe aus. Die Lösung wird anschließend auf 0-5 °C abgekühlt und das Produkt durch Filtration isoliert. Um noch anhaftende Reste an Mutterlauge zu entfernen, wird der Filterkuchen vorteilhaft noch mit einer Lösung von Sulfosalicylsäure oder einer gesättigten Kochsalzlösung nachgewaschen.

Die erfindungsgemäße Verbindung eignet sich besonders zum Einsatz als aktive Komponente in lichtempfindlichen Diazotypiereproduktionsmaterialien.

Die Erfindung ist deshalb auch gerichtet auf die Verwendung der lichtempfindlichen Diazoniumverbindung der allgemeinen Formel I in lichtempfindlichen Diazotypiematerialien.

Diazotypiematerialien sind als Ein- oder Zweikomponentenmaterialien bekannt. Sie bestehen im wesentlichen aus mindestens einem Diazoniumsalz und den üblichen Zusätzen für Einkomponentenmaterial oder aus mindestens einem Diazoniumsalz, der Kupplungskomponente, sauren Stabilisatoren und den üblichen Zusätzen für Zweikomponentenmaterialien.

Die Entwicklung erfolgt nach bildweiser Belichtung entweder mit einem alkalischen Entwickler der die Kupplungskomponente enthält oder mit einem alkalischen Entwickler allein.

Als Kupplungskomponenten können beispielsweise eingesetzt werden: Dihydroxynaphthaline, Dihydroxynaphthalinmono- und disulfonsäuren sowie deren Amide und substituierte Amide, a- und b-Hydroxynaphthoesäureamide und entsprechend substituierte Amide, Resorcin und seine Halogen-und Alkyl- oder Alkoxy-Derivate, Resorcylsäuren, gegebenenfalls mit Halogen substituiert, Resorcylsäureamide und substituierte Amide, Verbindungen mit aktiven Methylengruppen, wie Acetoacetyl- und Cyanoacetyl-Derivate, Mono-und Polyhydroxy-diphenyle, Polyhydroxy-diphenylsulfide, Aminophenol-Derivate, Pyrazolon-Derivate u.a.

Als Zusatz zu den Diazobeschichtungsmassen können bekannte Verbindungen verwendet werden, wie zum Beispiel Säurestabilisatoren, wie Citronensäure, Weinsäure, Borsäure, Sulfosalicylsäure, p-Toluolsulfosäure u.a. oder deren Gemische, Verbindungen zur Kontraststeigerung, wie Zinkchlorid, Aluminiumsulfat oder Nickelsulfat, Antioxydantien, wie Thioharnstoff oder Thioharnstoffderivate und Farbstoffe in kleiner Konzentration, wie Methylviolett, Alizarinirisol u.a., zur Stabilisierung und Verbesserung des ausbelichteten Hintergrundes, Entwicklungsbeschleuniger, wie Glycerin, Glycerinmono-, -di- und -triacetat, Harnstoff und alkylsubstituierte Harnstoffe u.a., feinteiliges oder kolloidales Siliziumdioxid oder Aluminiumoxid oder/und wäßrige Dispersionen oder kolloidale Lösungen organischer filmbildender Bindemittel, wie Polyvinylalkohol, Hydroxyethylcellulose, Methylcellulose u.a. oder latexartige Dispersionen von Polyvinylacetat, Polyvinylchlorid, Polyvinylchloridacetat, Polyvinylidenchlorid, Polyacrylnitril oder Polymethylmethacrylat.

Bei Verwendung von Kunststoffolien als Diazotypiematerialschichtträger ist es vorteilhaft, die Diazobeschichtungsmasse aus einem organischen Medium, das ein filmbildendes Bindemittel gelöst enthält, auf die Folienoberfläche aufzubringen. Geeignete filmbildende Bindemittel sind Celluloseether, wie Ethylcellulose, Celluloseester, wie Celluloseacetat, -acetopropionat, -butyrat, -acetobutyrat, Vinylpolymerisate, wie Polyvinylacetat, Polyvinylchlorid, Polyvinylidenchlorid, Vinylacetatmischpolymerisate, Poly-(methylmethacrylat)-Mischpolymerisate von Alkylacrylaten und Acrylsäure oder Polyphenylenoxide oder Ethylenglykol/Isophthalsäure/Terephthalsäure-Terpolymerisate.

Die Mengenverhältnisse der einzelnen Komponenten der Diazotypiematerialien können denen in den bisher zur Herstellung lichtempfindlicher Diazozusammensetzungen verwendeten, entsprechen. Diese Verhältnisse sind allgemein bekannt, so z.B. aus Kosar, Light-sensitive Systems, Wiley-Verlag, New York 1965,

Seiten 292-296.

Die erfindungsgemäßen Zusammensetzungen kann man dadurch herstellen, daß man die einzelnen Komponenten zusammen in einem geeigneten Reaktionsgefäß vorlegt. Sie werden vorzugsweise in wäßrigem Medium hergestellt, damit sie als wässriges Überzugsgemisch verwendet werden können.

Als Schichtträger eignen sich alle üblichen opaken und transparenten Materialien, z.B. beschichtete oder unbeschichtete opake oder transparente Papiere, Celluloseester, wie Cellulose-2-1/2-acetat und Cellulosetriacetat acetat, Polyester, wie Polyethylenterephthalat, Vinylpolymere, wie Polyvinylacetat oder Polystyrol sowie Polymere, wie Polyethylen oder Polypropylen.

Die Verarbeitung des Diazotypiematerials erfolgt, wie üblich, durch bildmäßige Belichtung unter einer transparenten Vorlage mit einer an ultravioletter und kurzwelliger sichtbarer Strahlung reichen Lichtquelle, beispielsweise mit einer Quecksilberhochdrucklampe oder einer Fluoreszenz-Leuchtstofflampe und anschließende Entwicklung, z.B. mit feuchtem oder trockenem Ammoniakgas bei normaler oder erhöhter Temperatur.

Die folgenden Beispiele sollen die Erfindung näher erläutern.

Beispiel 1: 4-N,N-Dimethylaminobenzoldiazonium-sulfosali-cylat

(Herstellung in Schwefelsäure)

188 ml (1,34 Mol) 50 %ige Schwefelsäure wurden mit 1600 ml Wasser vermischt und in dieser verdünnten Säure 136,0 g (1 Mol) 4-N,N-Dimethylaminoanilin gelöst. Die Lösung wurde einer Klärfiltration mit 20 g Aktivkohle unterzogen und anschließend bei 25 °C mit 131 ml (0,99 Mol) 40 %iger Natriumnitritlösung innerhalb von 10 Minuten diazotiert. Die Temperatur stieg dabei auf 39 °C an. Die entstandene Lösung des Diazoniumsulfats wurde anschließend einer weiteren Klärfiltration mit 14 g Aktivkohle unterworfen und dann in 30 Minuten auf 25 °C abgekühlt. Anschließend wurden innerhalb einer Minute 266,9 g 5-Sulfosalicylsäure x 2 $H_2O$ (1,05 Mol) zugegeben und schließlich wurde während 1,5 Stunden auf 4 °C abgekühlt. Das ausgefallene 4-N,N-Dimethylaminobenzoldiazonium-sulfosalicylat wurde über eine Nutsche abgesaugt und bei 30 °C im Umluftschrank getrocknet.

Ausbeute: 329,0 g (90,1 % d.Th.)

Reingehalt: 100 % (über Diazostickstoff)

| Elementaranalyse: $C_{15}H_{15}N_3O_6S$ (M = 365,4) | | | | |
|---|---|---|---|---|
| ber. | C 49,31 | H 4,14 | N 11,50 | S 8,78 |
| gef. | C 48,5 | H 4,1 | N 11,3 | S 9,1 |

Beispiel 2: 4-N,N-Dimethylaminobenzoldiazonium-sulfosalicylat

(Herstellung in Salzsäure)

235 ml (2,38 Mol) 30 %ige Salzsäure wurden mit 1260 ml Wasser versetzt und in dieser verdünnten Säure 50,0 g (0,37 Mol) 4-N,N-Dimethylaminoanilin gelöst. Die Lösung wurde einer Klärfiltration mit 7,5 g Aktivkohle unterzogen und anschließend bei 21 °C mit 48 ml (0,36 Mol) 40 %iger Natriumnitritlösung innerhalb von 4 Minuten diazotiert. Die Temperatur stieg dabei auf 29 °C an. Die entstandene Lösung des Diazoniumchlorids wurde anschließend einer weiteren Klärfiltration mit 5 g Aktivkohle unterworfen und dann in 30 Minuten auf 23 °C abgekühlt. Anschließend wurden innerhalb einer Minute 201,5 g (0,79 Mol) 5-Sulfosalicylsäure x 2 $H_2O$ zugegeben und das Ganze wurde während 2 Stunden auf 5 °C abgekühlt. Das ausgefallene 4-N,N-Dimethylaminobenzoldiazonium-sulfosalicylat wurde dann über eine Nutsche abgesaugt und im Umluftschrank getrocknet.

Ausbeute: 118,8 g (88,4 % d.Th.)

Reingehalt: 100 % (über Diazostickstoff)

Vergleichsbeispiel: 4-N,N-Dimethylaminobenzoldiazoniumchlorzinkat

37 ml (0,324 Mol) 65 %ige Zinkchloridlösung wurden mit 95 ml Wasser verdünnt und dann mit 50,6 ml (0,478 Mol) 30 %iger Salzsäure versetzt sowie 10,0,g (0,074 Mol) 4-N,N-Dimethylaminoanilin zugegeben. Die entstandene Lösung wurde einer Klärfiltration mit 1,5 g Aktivkohle unterzogen und anschließend bei 30 °C mit 9,6 ml (0,072 Mol) 40 %iger Natriumnitritlösung innerhalb von 2 Minuten diazotiert. Die Temperatur stieg dabei auf 34 °C an. Die entstandene Lösung des Diazoniumsalzes wurde anschließend einer weiteren Klärfiltration mit 1 g Aktivkohle unterworfen und dann bei 23 °C mit 75 g (1,282 Mol) Kochsalz versetzt. Die Lösung wurde dann eine Stunde auf 5 °C abgekühlt und das ausgefallene Zinkchlorid stabilisierte 4-N,N-Dimethylaminobenzoldiazoniumchlorid abgesaugt und bei 30 °C im Umluftschrank getrocknet.
Ausbeute: 20,6 g (78,7 %)
Reingehalt: 97 % (photometrisch)

Beispiel 3: 4-N,N-Diethylaminobenzoldiazonium-sulfosalicylat

210 ml (0,213 Mol) 30 %ige Salzsäure wurden mit 1150 ml Wasser verdünnt und hierin wurden anschließend 50 g (0,30 Mol) 4-N,N-Diethylaminoanilin gelöst. Die Lösung wurde mit 8 g Aktivkohle geklärt und anschließend bei 25 °C mit 39 ml (0,29 Mol) 40 %iger Natriumnitritlösung innerhalb von 5 Minuten diazotiert. Die Temperatur stieg dabei auf 30 °C an. Die entstandene Lösung des Diazoniumsalzes wurde anschließend einer weiteren Klärfiltration mit 5 g Aktivkohle unterworfen und dann auf 20 °C abgekühlt. Anschließend wurden innerhalb von 3 Minuten 280,0 g (0,33 Mol)einer 30 %igen Lösung von 5-Sulfosalicylsäure x 2 $H_2O$ zugegeben und dann wurde während zwei Stunden auf 5 °C abgekühlt. Das ausgefallene 4-N,N-Diethylaminobenzoldiazonium-sulfosalicylat wurde über eine Nutsche isoliert und bei 30 °C getrocknet.
Ausbeute: 107,4 g (89,1 % d.Th.)
Reingehalt: 98 % (über Diazostickstoff)

Beispiel 4: 3-Methyl-4-N-pyrrolidinobenzoldiazonium-sulfosalicylat

405,3 g (2,3 Mol) 3-Methyl-4-pyrrolidinoanilin wurden in 2,88 l 11,5 %iger Salzsäure (10,2 Mol) gelöst und die Lösung mit 30 g Aktivkohle geklärt. Anschließend diazotierte man innerhalb von 70 Minuten mit 306 ml (2,3 Mol) 40 %iger Natriumnitritlösung bei 8 - 10 °C. Man rührte noch 30 Minuten nach und ließ die Temperatur des Reaktionsgemisches in dieser Zeit auf 20 °C ansteigen. Die entstandene Lösung des Diazoniumsalzes wurde einer weiteren Klärfiltration mit 30 g Aktivkohle unterworfen und anschließend mit 614 g (2,4 Mol) 5-Sulfosalicylsäure x $2H_2O$ gelöst in 1,15 l Wasser versetzt. Zur Vervollständigung der Fällung wurde auf 0 °C abgekühlt und zwei Stunden nachgerührt. Das ausgefallene 3-Methyl-4-N-pyrrolidinobenzoldiazonium-sulfosalicylat wurde über eine Nutsche isoliert und bei 40 °C im Umlufttrockenschrank getrocknet.
Ausbeute: 828 g (88,2 % d. Th.)
Reingehalt: 99,3 % (über Diazostickstoff)

Beispiel 5L:

Ein für das Diazotypieverfahren geeigneter Papierträger wurde mit einem Vorstrich beschichtet. Dieser bestand aus einer wäßrigen $SiO_2$-Suspension und einem Bindemittelsystem, so zum Beispiel einer Polyvinylacetatemulsion oder einer wäßrigen Lösung von Natriumcasinat.

Der so beschichtete Papierträger wurde mit einer Flüssigkeit mit folgenden Komponenten sensibilisiert:

| | |
|---|---|
| Citronensäure | 200 g |
| Coffein | 150 g |
| Thioharnstoff | 500 g |
| 2,2',4,4'-Tetrahydroxydiphenyl | 50 g |
| 2,7-Dihydroxy-3,6-naphthalin-disulfonsäure | 110 g |
| 4-N,N-Dimethylaminobenzoldiazonium-sulfosalicylat | 175 g |
| Zinkchlorid | 500 g |
| Wasser | 10.000 $cm^3$ |

Das getrocknete, sensibilisierte Material wurde unter einer Vorlage belichtet und anschließend mittels Ammoniak entwickelt. An den Bildstellen erhielt man einen tiefschwarzen Farbton, der sich in keiner Weise von dem Farbton unterscheidet, den man bei Verwendung derselben sensibilisierten Flüssigkeit, jedoch mit vergleichbarer molarer Menge an 4-N,N-Dimethylaminobenzoldiazoniumchlorzinkat anstelle des entsprechenden Sulfosalicylats erhielt. Weiterhin treten keine Unterschiede in den anwendungstechnischen Eigenschaften wie Entwicklungsgeschwindigkeit und Thermostabilität der Diazotypiepapiere auf.

Beispiel 6:

Ein wie in Beispiel 5 vorbehandelter Papierträger wurde mit einer Flüssigkeit aus folgenden Bestandteilen sensibilisiert:

| | |
|---|---|
| Citronensäure | 200 g |
| Thioharnstoff | 600 g |
| Coffein | 150 g |
| Isopropanol | 100 $cm^3$ |
| 2,3-Dihydroxynaphthalin-6-sulfonsäure (Na-Salz) | 50 g |
| 4-N,N-Dimethylaminobenzoldiazonium-sulfosalicylat | 110 g |
| Zinkchlorid | 500 g |
| Saponin | 2 g |
| Wasser | 10.000 $cm^3$ |

Nach Trocknen wurde das Material an einer Lichtpausmaschine durch eine Vorlage hindurch belichtet. Anschließend erfolgte die Entwicklung des belichteten Materials mittels Ammoniak. Man erhielt von der Vorlage eine positive, tiefblaue Kopie. Bei Verwendung von 4-N,N-Dimethylaminobenzoldiazonium-chlorzinkat anstelle von 4-N,N-Dimethylaminobenzoldiazonium-sulfosalicylat traten keine Farb- und Kontrastunterschiede im Vollton auf.

Beispiel 7:

Ein wie in Beispiel 5 vorbehandeltes Papier wurde mit einer Flüssigkeit folgender Zusammensetzung sensibilisiert:

| | |
|---|---|
| Citronensäure | 200 g |
| Coffein | 150 g |
| Thioharnstoff | 600 g |
| 2,3-Dihydroxynaphthalin | 78 g |
| 4-N,N-Diethylaminobenzoldiazonium-sulfosalicylat | 188 g |
| Zinkchlorid | 500 g |
| Saponin | 2 g |
| Isopropanol | 100 $cm^3$ |
| Wasser | 10.000 $cm^3$ |

Das sensibilisierte Material wurde belichtet und mittels Ammoniak entwickelt. Die unbelichteten Bildstellen waren tiefblau.

Bei molarem Ersatz von 4-N,N-Diethylaminobenzoldiazonium-sulfosalicylat durch 4-N,N-Diethylaminobenzoldiazonium-chlorzinkat in der zuvor genannten Rezeptur trat die anlaoge Farbreaktion auf.

Beispiel 8:

Ein transparentes Pauspapier mit einem Flächengewicht von 80 g/m$^2$ wurde einseitig mit Celluloseacetobutyrat versehen und danach die lackierte Seite sensibilisiert. Die Sensibilisierungslösung besaß folgende Zusammensetzung:

900 ml Isopropanol
100 ml Weichwasser
100 ml Ameisensäure (100 %ig)
35 g Sulfosalicylsäure
40 g Resorcin
14 g 3-(2′-Hydroxyethyl)phenol
7 g 2-Hydroxynaphthalin-3-carbonsäure-N-(2′-hydroxyethyl)amid
20 g 4-N-Pyrrolidino-3-methylbenzoldiazonium-sulfosalicylat

Das auf diese Weise erhaltene lichtempfindliche Transparentpapier wurde durch eine transparente Vorlage belichtet und mit Ammoniak entwickelt. An den Bildstellen entstand ein tiefbrauner Farbton. Diese Kopie war als Zwischenoriginal für weitere Pausen geeignet.

Ersetzte man in der Sensibilisierungslösung das 4-N-Pyrrolidino-3-methylbenzoldiazonium-sulfosalicylat durch das entsprechende Zinksalz, so erhielt man ein transparentes Lichtpausmaterial mit identischen Eigenschaften.

## Ansprüche

1. Lichtempfindliche Diazoniumverbindung der allgemeinen Formel I

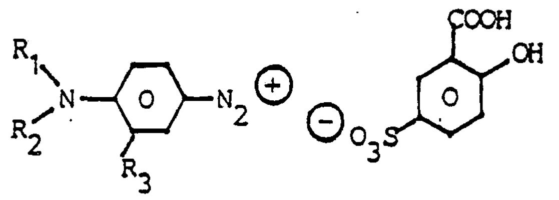

I

in der $R_1$ und $R_2$ gleich oder verschieden sind und
$R_1$ Methyl, Ethyl oder 2-Hydroxyethyl und
$R_2$ Methyl oder Ethyl oder
$R_1$ und $R_2$ gemeinsam eine $(CH_2)_n$ Gruppierung über dem Stickstoffatom, an das sie gebunden sind, mit n gleich 4 oder 5 und
$R_3$ Wasserstoff oder Methyl
bedeuten.

2. Verbindung nach Anspruch 1, in der $R_1$ und $R_2$ Methyl und $R_3$ Wasserstoff bedeuten.

3. Verbindung nach Anspruch 1, in der $R_1$ und $R_2$ zusammen $-(CH_2)_4-$ und $R_3$ Methyl bedeuten.

4. Verfahren zur Herstellung der Diazoniumverbindung der allgemeinen Formel I nach Ansprüchen 1 bis 3, dadurch gekennzeichnet, daß man eine Verbindung der allgemeinen Formel II

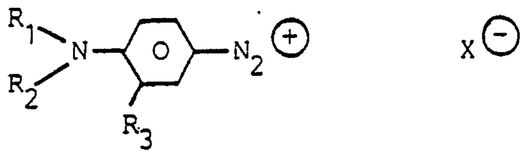

II

worin $R_1$, $R_2$ und $R_3$ die angegebenen Bedeutungen haben und X das Anion einer starken Säure ist, mit

einer Verbindung der allgemeinen Formel III

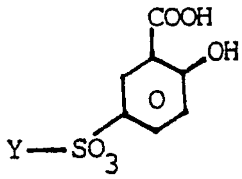

III

wobei Y ausgewählt ist aus der Gruppe Wasserstoff und Salz bildendes Metallion, in an sich bekannter Weise umsetzt und isoliert.

5. Verwendung der lichtempfindlichen Diazoniumverbindung der allgemeinen Formel I nach Ansprüchen 1 bis 3 in lichtempfindlichen Diazotypiematerialien.